(19) Europäisches Patentamt

European Patent Office

Office européen des brevets

(11) Publication number: 0 504 869 A2

# EUROPEAN PATENT APPLICATION

(21) Application number: 92104774.2

(22) Date of filing: 19.03.92

(51) Int. Cl.⁵: C12N 15/82, C12N 15/11, A01H 5/00, A01N 63/00

(30) Priority: 20.03.91 JP 81544/91

(43) Date of publication of application:
23.09.92 Bulletin 92/39

(84) Designated Contracting States:
FR IT

(71) Applicant: JAPAN TOBACCO INC.
12-16, Higashi Shinagawa 4-chome,
Shinagawa-ku, Tokyo 140(JP)

(72) Inventor: Saito, Yasuhito c/o Japan Tobacco
Inc.
Higashibara 700, Toyoda-cho,
Iwata-gun, Shizuoka, 438(JP)
Inventor: Komari, Toshihiko c/o Japan
Tobacco Inc.
Higashibara 700, Toyoda-cho,
Iwata-gun, Shizuoka, 438(JP)
Inventor: Kumashiro, Takashi c/o Japan
Tobacco Inc.
Higashibara 700, Toyoda-cho,
Iwata-gun, Shizuoka, 438(JP)
Inventor: Masuta, Chikara c/o Life Science
Research Lab.
6-2, Umegaoka, Midori-ku, Yokohama-shi,
Kanagawa, 227(JP)
Inventor: Hayashi, Yumiko c/o Life Science
Research Lab.
6-2, Umegaoka, Midori-ku, Yokohama-shi,
Kanagawa, 227(JP)
Inventor: Takanami, Yoichi c/o Life Science
Research Lab.
6-2, Umegaoka, Midori-ku, Yokohama-shi,
Kanagawa, 227(JP)

(74) Representative: Reinhard, Skuhra, Weise
Friedrichstrasse 31
W-8000 München 40(DE)

(54) Tomato resistant to cucumber mosaic virus and method for transforming tomato.

(57) A tomato resistant to cucumber mosaic virus and a method for transforming tomatoes are disclosed. The tomato has a cDNA of a satellite RNA of cucumber mosaic virus. The method for transforming a tomato according to the present invention comprises transforming the tomato with Agrobacterium tumefaciens to which a plasmid containing a DNA region originating from the virulence region of Ti plasmid pTiBo542, and a cDNA of a satellite RNA of cucumber mosaic virus are introduced.

Fig. 1

ABBREVIATION : NPT : neomycin phosphotransferase, Ori : replication origin of ColE1

Cos : Cos site of λ phage, BR : border of right, BL : border of left,

TC : tetracycline-resistant gene, SPEC : spetinomycin-resistant gene,

Nos : promoter of nopaline synthase, 73 : cDNA of 73-satRNA,

AP : ampicillin-resistant gene, B : Bam HI, K : Kpn I, S : Sal I, Sa : Sac II,

E : Eco RI, P : Pst I

## BACKGROUND OF THE INVENTION

### I. Field of the Invention

This invention relates to a tomato resistant to cucumber mosaic virus and to a method for transforming a tomato so as make the tomato resistant to cucumber mosaic virus.

### II. Description of the Related Art

Cucumber mosaic virus (hereinafter also referred to as "CMV" for short) is an important pathogenic virus with which a wide variety of plants are infected. However, since no cultivated tomato varieties or no wild type tomatoes are resistant to cucumber mosaic virus, only the indirect methods for the prevention of infection of this virus, such as methods of cultivation and application of agricultural chemicals, are now employed.

Recently, the development of genetic recombination technique has made it possible for a plant to acquire resistance to a virus by the transformation by which a gene encoding the coat protein of a virus or a gene encoding satellite RNA is introduced.

It is known that if an avirulent virus is preliminarily inoculated to a plant, even if the plant is later infected with a highly virulent virus of the same species as the preliminarily inoculated avirulent virus, the severity of the disease caused by the highly virulent virus is attenuated. It is thought that this interference action is due to the fact that the preliminarily inoculated virus prevents the uncoating of the later infected virus. It is thus known that by introducing a gene encoding the coat protein of a virus into a plant by a transformation method, the plant acquires the resistance to the virus according to the similar mechanism to the above-mentioned interference action. For example, the attenuation of diseases has been reported for tobacco mosaic virus (Powell et al., Science 232:738-743, 1986), alfalfa mosaic virus (Tumer et al., EMBO J. 6:1181-1188, 1987; and Loesch-Fries et al., EMBO J. 6: 1845-1851, 1987), potato virus X (Hemenway et al., EMBO J. 7:1273-1280, 1988), potato virus Y (Lawson et al., Bio/Technology 8:127-134, 1990) and cucumber mosaic virus (Cuozzo et al., Bio/Technology 6:549-558, 1988).

Cucumber mosaic virus is an RNA virus. The virus particle normally contains four kinds of RNAs (i.e., RNA1, RNA2, RNA3 and RNA4). RNA1 and RNA2 have genes concerning the replication of the RNA virus. The gene on the 5' region of RNA3 is thought to take part in the intercellular transfer of the virus. RNA4 is the same as the gene on the 3' region of RNA3 and has a gene of the coat protein of the virus. However, some cucumber mosaic virus isolates have an additional RNA called satellite RNA. This satellite RNA is a low molecular weight RNA with a size of 330 - 390 bases. The satellite RNA per se does not have an ability to replicate and the replication thereof is dependent on the parent virus. An important characteristic of the satellite RNA is that it changes the symptoms of the infected plants. That is, when cucumber mosaic virus and a satellite RNA are simultaneously replicated in a host plant, the replication and accumulation of the virus in the host are often inhibited so that the symptoms caused by the virus are attenuated. Thus, it was tried to introduce the satellite RNA into a plant by an appropriate transformation method so as to express the satellite RNA in the plant, thereby inhibiting the replication of cucumber mosaic virus and the development of the disease. The attenuation of the symptoms has been reported in tobacco (Harrison et al., Nature 328:799-802, 1987; and Japanese Laid-open Patent Application (Kokai) No. 64-27474). Although a tomato having a satellite RNA introduced therein was produced, the symptoms were severer than in the normal tomatoes so that it was failed to give to a tomato the resistance to cucumber mosaic virus (McGarvey et al., Biochem. and Biophys. Res. Commun. 170:548-555, 1990; Tousch et al., C.R. Acad. Sci. Paris 311, Serie III:377-384, 1990).

Thus, nobody has succeeded in producing a tomato which exhibits a practically sufficient resistance to cucumber mosaic virus by introducing a satellite RNA into the tomato.

## SUMMARY OF THE INVENTION

Accordingly, an object of the present invention is to provide a tomato which has excellent resistance to cucumber mosaic virus.

Another object of the present invention is to provide a method for transforming a tomato so as to give to the tomato the resistance to cucumber mosaic virus.

The present inventors intensively studied the satellite RNAs of cucumber mosaic virus. As a result, the present inventors succeeded in isolating a satellite RNA which has an extremely high symptoms-attenuating effect and which does not show adverse effects, such as changing of the color of the tomato to yellow and aggravation of the symptoms, and also succeeded in producing tomatoes highly resistant to cucumber mosaic virus by introducing this satellite RNA to the tomatoes, thereby completing the present invention.

That is, the present invention provides a tomato resistant to cucumber mosaic virus, which has a cDNA of a satellite RNA of cucumber mosaic virus.

The present invention also provides a method for transforming a tomato comprising transforming said tomato with Agrobacterium tumefaciens to which a plasmid containing a DNA region originating from the virulence region of Ti plasmid pTiBo542, and a cDNA of a satellite RNA of cucumber mosaic virus are introduced.

By the present invention, tomatoes with practically sufficient resistance to cucumber mosaic virus, which are not suffered from the delay in growing or the reduction of the yield of fruits, as well as a method for producing the tomatoes were provided. The tomato of the present invention is a CMV-resistant tomato which substantially does not exhibit the symptoms even when infected with cucumber mosaic virus ordinary strain (O strain).

BRIEF DESCRIPTION OF THE DRAWINGS

Fig. 1 is a view for explaining the construction of plasmid pYS143;

Fig. 2 shows the complete base sequence of T73-satRNA;

Fig. 3 schematically shows the results of the Southern blotting analysis of the transformants Sat-71 and Sat-72;

Fig. 4 schematically shows the results of the Northern blotting analysis of the transformants Sat-71 and Sat-72; and

Fig. 5 schematically shows the results of the agarose gel electrophoresis of the RNAs of the transformant Sat-71 inoculated with cucumber mosaic virus.

DETAILED DESCRIPTION OF THE PREFERRED EMBODIMENTS

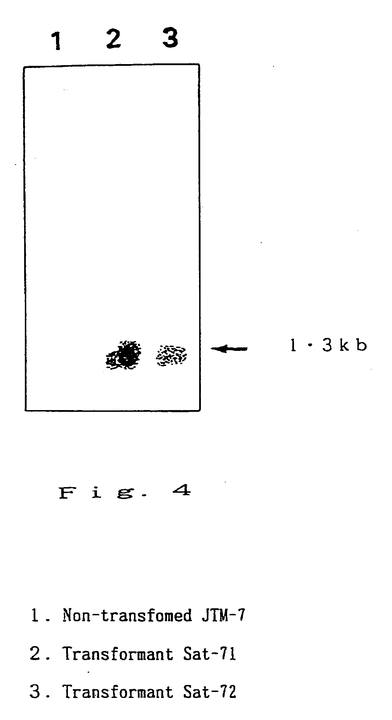

In the present invention, it is necessary to use a satellite RNA of cucumber mosaic virus, which has a high symptoms-attenuating effect for tomatoes and which hardly exhibits adverse effects in tomatoes. The present inventors collected the plants infected with cucumber mosaic virus, which exhibit mild symptoms. From the collected plants, four kinds of satellite RNAs were isolated. These satellite RNAs were named S19-satRNA, T43-satRNA, T62-satRNA and T73-satRNA, respectively. By inoculating these satellite RNAs to tomatoes, it was found that T73-satRNA exhibits the highest symptoms-attenuating effect (Masuta et al., Ann. Phytopath. Soc. Japan 56:207-212, 1990). The base sequence of the T73-satRNA is shown in Fig. 2, and the base sequences of the above-mentioned four kinds of satellite RNAs are described in Masuta et al., supra. It should be noted that the satellite RNAs which may be employed in the present invention are not restricted to those mentioned above. A satellite RNA which may be employed in the present invention may be obtained by infecting a plant such as tobacco with cucumber mosaic virus, which plant exhibits symptoms upon being infected with cucumber mosaic virus, extracting the satellite RNA by a conventional method from the plant exhibiting attenuated symptoms, infecting tomatoes with the thus obtained satellite RNA and cucumber mosaic virus, and selecting the satellite RNA which gives an excellent resistance to the cucumber mosaic virus.

The method for producing the CMV-resistant tomato according to the present invention will now be described.

In order to introduce the satellite RNA and to express it in a plant, it is necessary to convert the satellite RNA to cDNA. Therefore, a cDNA containing a region corresponding to the full length of the satellite RNA is cloned by a conventional method employing a synthetic primer and a reverse transcriptase.

The cDNA of the satellite RNA thus obtained may be introduced into tomatoes by transformation method. The efficiency of the transformation of tomatoes is hitherto low, and for some varieties, the transformation is extremely difficult. The present inventors developed a novel method for transformation of tomatoes which has very high efficiency and which does not damage the tomatoes. The cDNA of the cucumber mosaic virus satellite RNA may be introduced into tomatoes by this method.

In this method, tomatoes are transformed with Agrobacterium tumefaciens in which a plasmid containing a DNA region originating from the virulence region of a Ti plasmid pTiBo542 (Jin et al., Journal of Bacteriology 169:4417-4425, 1987) and a cDNA of the satellite RNA of cucumber mosaic virus are introduced. The DNA region originating from the virulence region of pTiBo542 may preferably contain virB, virG and virC genes. A preferred example of such a plasmid is the plasmid pTOK162 which will be described in detailed in the examples hereinbelow described. The cDNA of the satellite RNA of cucumber mosaic virus may be inserted into the T region of the plasmid. Although this insertion may be attained by a conventional subcloning technique employing restriction enzymes, in some cases, it is not easy to insert the desired DNA in the T region of a large plasmid having a number of restriction sites such as plasmid pTOK162. In such a case, the desired DNA can be inserted in the T region of pTOK162 by utilizing the in vivo homologous recombination (Herrera-Esterella et al, EMBO J. 2:987-995, 1983; Horch et al., Science, 223:496-498, 1984) in the cells of Agrobacterium tumefaciens. That is, pTOK162 is preliminarily introduced into Agrobacterium tumefaciens and the plasmid pBR322 (or a similar plasmid) containing the cDNA of the satellite RNA is further introduced into Agrobacterium tumefaciens. Since pTOK162 has a region homologous with a region of pBR322, the pBR322 derivative containing the cDNA of the satellite RNA is inserted into pTOK162 by the genetic recombination via the homologous regions. Unlike pTOK162, pBR322 cannot replicate by itself in Agrobacterium tumefaciens. Therefore, pBR322 can only be alive in Agrobacterium tumefaciens in the inserted form in pTOK162 (the recombined pTOK162 and pBR322 is hereinafter designated "pTOK162::pBR322"). By selecting the transformants based on the selection markers (such as drug resistance) specific to each of pTOK162 and pBR322 derivative, Agrobacterium tumefaciens transformants containing pTOK162::pBR322 may be obtained.

By culturing the Agrobacterium tumefaciens to which pTOK162::pBR322 is introduced together with fragments of cotyledon of a tomato, the cDNA of the satellite RNA may be introduced into the tomato. After washing the cotyledon fragments, the transformed plants may be selected based on the selection marker. That is, if the selection marker is a drug resistance, the transformants may be obtained by placing the cotyledon fragments on a stem and leaf-differentiation medium containing the drug and then placing the plants resistant to the drug on a rooting medium.

A tomato resistant to cucumber mosaic virus may be selected by checking the morphologies and characteristics of the thus obtained transformants, confirming the introduction of the cDNA of the satellite RNA and transcription of the mRNA therefrom and by actually inoculating cucumber mosaic virus to check the resistance.

The present invention will now be described by way of examples thereof. It should be noted, however, the examples are presented for the illustration purpose only and should not be interpreted in any restrictive way.

In the following examples, unless otherwise specified, the operations were carried out in accordance with the methods described in T. Maniatis, Molecular Cloning, Cold Spring Harbor (1982)).

(1) Isolation of T73-satRNA and Cloning of cDNA Thereof

Various cucumber mosaic viruses were collected mainly from tobacco fields in Tochigi Prefecture, Japan and four kinds of satellite RNAs of cucumber mosaic viruses were isolated. These four kinds of satellite RNAs were designated S19-satRNA, T43-satRNA, T62-satRNA and T73-satRNA, respectively. These satellite RNAs of cucumber mosaic viruses were inoculated to tomatoes to find that T73-satRNA especially excels in attenuating the disease in tomatoes (Masuta et al.,

supra).

A cDNA containing the full length of T73-satRNA was cloned using a synthetic primer and a reverse transcriptase. This operation was carried out as follows: Using a synthetic probe which is complementary to the 3'-terminal region of T73-satRNA as a primer, the first strand of the cDNA was synthesized to obtain an RNA-DNA hybrid. After treating this hybrid with RNaseH, the second DNA strand was formed by using a synthetic probe which is complementary to the 3'-terminal region of the thus produced DNA chain as a primer. The thus obtained full length cDNA was inserted in plasmid pUC119 (commercially available from Takara Shuzo Co., Ltd., Kyoto, Japan) at Sma I site.

(2) Insertion of Satellite RNA into Plasmid pTOK162

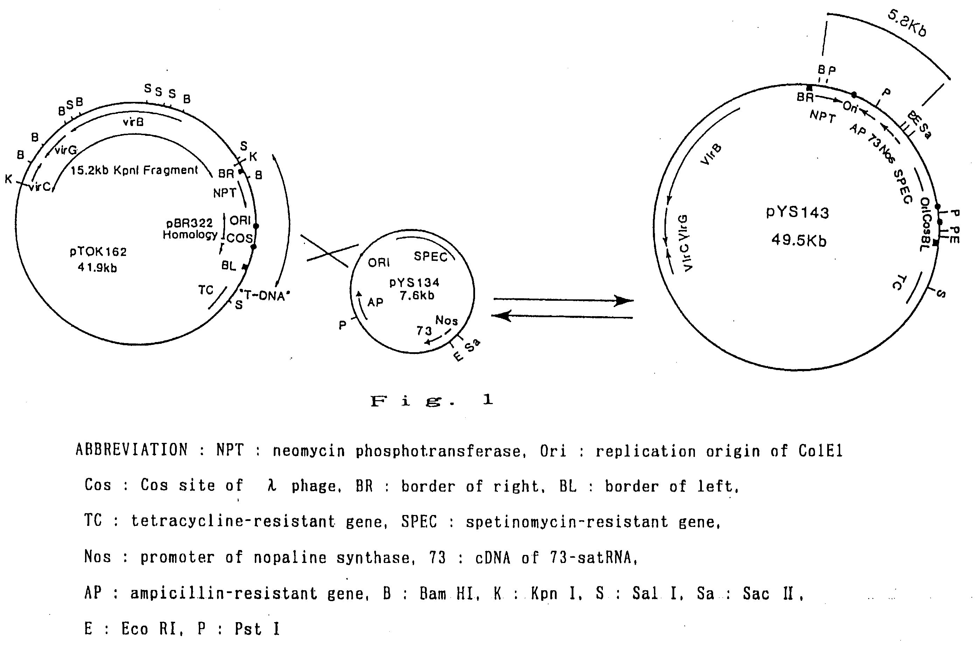

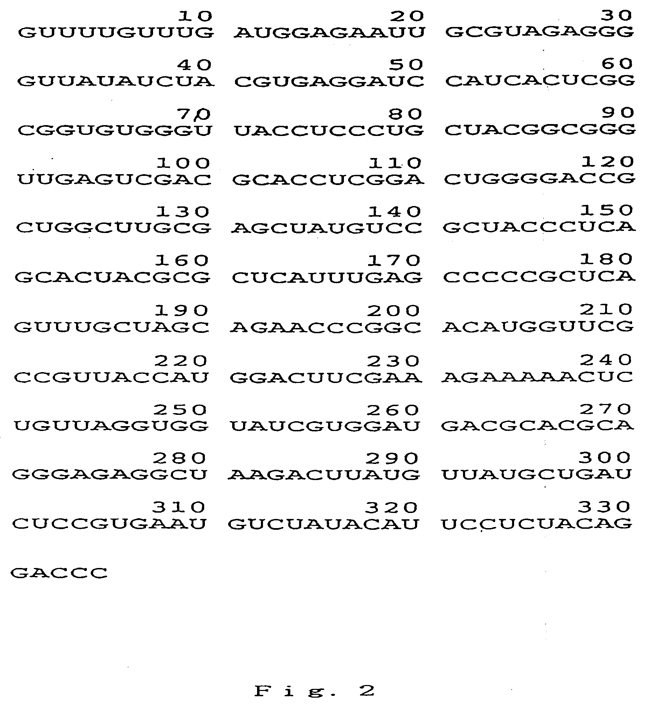

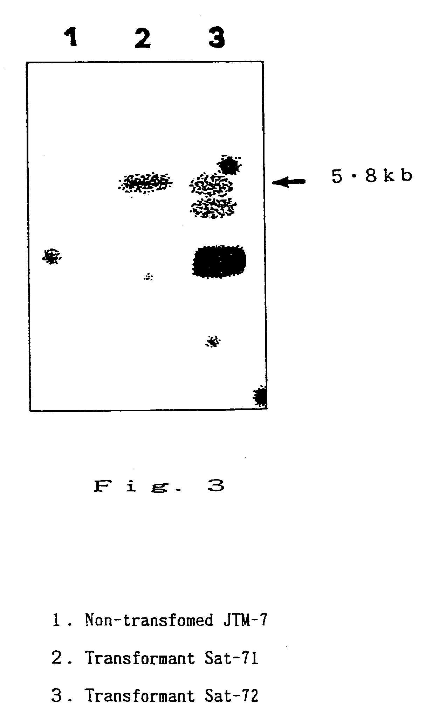

The cDNA obtained in (1) was inserted into an expression vector for plants pLGV2382 to obtain pLGV2382-73sat. This recombinant vector was digested with restriction enzymes Hin dIII and Pvu II and a DNA fragment containing Nos promoter, T73-satRNA and Nos terminator was recovered. This DNA fragment was treated with E. coli DNA polymerase I Klenow fragment to blunt the ends. On the other hand, pBR322 containing the spectinomycin resistant gene originated from 2.5 kb Cla I fragment of transposon Tn7 was digested with Cla I and the resulting fragment was blunted with E. coli DNA polymerase I Klenow fragment. The resultant was ligated with the above-mentioned blunted DNA fragment by T4 ligase to obtain a plasmid pYS134 containing the cDNA of T73-satRNA and the spectinomycin resistant gene. The thus obtained pYS134 was introduced into Agrobacterium tumefaciens LBA4404 (Hoekema et al., Nature 303:179-180, 1983) containing a plasmid pTOK162 (Komari, Plant Cell Report 9: 303-306, 1990) containing genes giving resistances to kanamycin and tetracycline by triple cross (Ditta et al., Proc. Natl. Acad. Sci. USA 77:7347-7351, 1980). Then colonies resistant to both kanamycin and spectinomycin were selected. The selected resistant colonies emerged due to the recombination between the plasmids pYS134 and pTOK162 at their homologous regions. By the operations described above, a plasmid having T73-satRNA inserted in plasmid pTOK162 was constructed. This plasmid was named pYS143. The process for constructing plasmid pYS143 is shown in Fig. 1.

(3) Transformation of Tomatoes

Tomatoes were transformed with the LBA4404-(pYS143) obtained in (2). As the tomatoes, variety Momotaro (commercially available from Takii Co., Ltd., Kyoto, Japan) and JTM-7 (grown in Plant Development Laboratories of Japan Tobacco, Inc.) were used. Seeds of these tomatoes were sterilized with ethanol and sodium hypochlorite, and then sown in a medium containing the inorganic salts of Linsmaier and Skoog medium (1965), 30 g/l of sucrose and 0.9% of agar. Ten days after the sowing, cotyledons of the tomatoes aseptically germinated were cut into pieces sizing 0.7 cm x 0.7 cm. One gram of the resulting fragments of the cotyledons and about $10^8$ cells of Agrobacterium tumefaciens were incubated together in a liquid medium containing the inorganic salts of Linsmaier and Skoog medium and 30 g/l of glucose for 40 hours at 23°C. The thus treated fragments of the cotyledons were washed with sterilized water to wash off bacteria and then the fragments were placed on an agar (0.9%) medium containing the inorganic salts of Linsmaier and Skoog medium, 0.3 mg/l of indole acetic acid, 10 mg/l of isopentenyladenine, 100 mg/l of kanamycin, 250 mg/l of cefotaxime and 30 g/l of glucose. Twenty days after the commencement of the culturing, kanamycin-resistant calli emerged. These calli were further cultured on the same medium for additional 15 days. As a result, stems and leaves showing resistance to kanamycin emerged. These stems and leaves were cut and the cut pieces were placed on an agar (0.9%) medium containing the inorganic salts of Linsmaier and Skoog medium, 30 g/l of sucrose and 250 mg/l of cefotaxime. Thirty days after the commencement of this culturing, the plants from which roots were grown were cultivated in a closed green house. As a result, four regenerated plants were obtained from JTM-7 (designated Sat-71, Sat-72, Sat-73 and Sat-74) and three regenerated plants were obtained from Momotaro (designated as Satm-11, Satm-12 and Satm-13).

(4) Morphologies of Regenerated Plants

The characteristics of the regenerated plants obtained in (3) and their parent varieties JTM-7 and Momotaro were compared. As a result, it was confirmed that the morphologies and the growth characteristics of the regenerated plants were the same as those of the parent varieties. Further, the fertilities and formations of fruits of the regenerated plants were normal as in the parent varieties.

(5) Confirmation of Introduction of T73-satRNA Gene by Southern Blotting Analysis

Among the regenerated plants, Sat-71, Sat-72, Satm-11, Satm-12 and Satm-13 were checked for the introduction of T73-satRNA gene by Southern blotting analysis. First, total DNAs were extracted from each of these five plants by the method of Komari et al (Komari et al., Theor. Appl. Genet. 77:547-552, 1989). The obtained total DNAs were digested with a restriction enzyme Bam HI and the resultant was subjected to agarose gel electrophoresis. The separated DNA fragments were transferred to a nylon membrane (Gene Screen Plus, commercially available from Dupont), and Southern blotting analysis was carried out using Hin dIII - Eco RI fragment of pUC119-73sat, which has a size of 0.4 kb and which was labelled with $^{32}$P by the random prime method (the kit is commercially available from Boehringer Mannheim).

The results are shown in Fig. 3. As shown in Fig. 3, a band corresponding to a size of 5.8 kb was detected in Sat-71 and Sat-72. This size substantially corresponds to the size of the Bam HI fragment containing the introduced T73-satRNA while such a band was not detected in the parent plant JTM-7 which was also analyzed as a control. From these results, it was confirmed that the cDNA of T73-SatRNA was introduced into these two plants.

On the other hand, the same analysis was carried out for the three transformants originated from Momotaro. As a result, the introduction of the cDNA of T73-satRNA was confirmed in all of the three transformants originated from Momotaro.

(6) Confirmation of Transcription of T73-satRNA by Northern Blotting Analysis

The regenerated plants Sat-71 and Sat-72 were checked for the transcription of mRNA of T73-satRNA gene. First, total RNAs were extracted from each of these two plants and the extracted RNAs were denatured by glyoxal and dimethylsulfoxide in accordance with the Thomas' method (Thomas, Proc. Natl. Acad. Sci. USA 77:5201-5205(1980)). The resultant was subjected to agarose gel electrophoresis. The separated RNAs were transferred to a nylon membrane (Gene Screen Plus (commercially available from Dupont) and Northern blotting analysis was carried out using the DNA fragment of 0.4 kb used in the above-described Southern blotting analysis as a probe.

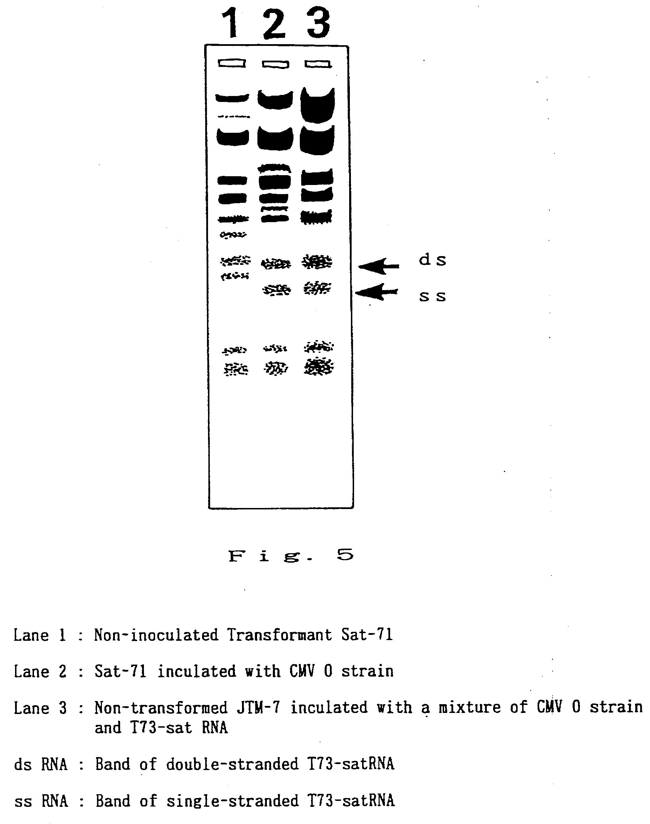

The results are shown in Fig. 4. As a result of the Northern blotting analysis, transcription of a mRNA of 1.3 kb was confirmed in Sat-71 and Sat-72. The size of this mRNA is identical to the expected mRNA transcribed from T73-satRNA. Such a transcription of mRNA was not observed in the parent JTM-7 used as a control. These results indicate that the mRNA is transcribed from T73-satRNA gene in these two plants.

The regenerated plants Satm-11, Satm-12 and Satm-13 were analyzed in the same way. As a result, the transcription of the mRNA of T73-satRNA gene was confirmed in these three plants.

(7) Expression of Symptoms by Mechanical Inoculation with Cucumber Mosaic Virus

Transformants Sat-71 and Sat-72, in which the introduction of the cDNA of T73-satRNA and the transcription of mRNA therefrom were confirmed were inoculated with ordinary strain (O strain) of cucumber mosaic virus. As a control, the parent variety JTM-7 was also inoculated with the virus.

As a result, although the mosaic-like symptom was slightly observed in the Sat-71 and Sat-72 inoculated with cucumber mosaic virus O strain, the morphologies and growth conditions of these transformants were the same as the parent JTM-7 which was not inoculated with the virus and the fruits were also normally formed as in the parent plant. In contrast, the growth of the parent JTM-7 inoculated with the virus was extremely delayed and no fruits were formed. From these results, it was proved that the expression of the symptoms in the plants in which the expression of T73-satRNA was confirmed is extremely slight even when the plants are infected with cucumber mosaic virus ordinary strain.

RNAs were extracted from Sat-71 and Sat-72 inoculated with cucumber mosaic virus O strain and were analyzed by agarose gel electrophoresis. The results are shown in Fig. 5. It was confirmed that T73-satRNA as well as the RNA of cucumber mosaic virus were replicated. On the other hand, T73-satRNA was not detected in the JTM-7 inoculated with cucumber mosaic virus O strain. From these results, it was proved that the replication of T73-satRNA observed in the transformants attenuates the symptoms.

Transformants Satm-11, Satm-12 and Satm-13 were also inoculated with cucumber mosaic virus O strain. As a result, the symptoms were substantially not observed. Further, the replication of T73-satRNA was observed in these transformants.

By the above-described experiments, it was confirmed that the tomatoes in which the cDNA of T73-satRNA only exhibit slight symptoms even if they are infected with cucumber mosaic virus, so that they acquired substantial resistance to the virus. Thus, by the present invention, production of tomatoes resistant to cucumber mosaic virus, which could not hitherto be attained, was first attained.

## Claims

1. A tomato resistant to cucumber mosaic virus, which has a cDNA of a satellite RNA of cucumber mosaic virus.

2. The tomato of claim 1, wherein said cDNA is a cDNA of T73-satRNA.

3. A method for transforming a tomato comprising transforming said tomato with Agrobacterium tumefaciens to which a plasmid containing a DNA region originating from the virulence region of Ti plasmid pTiBo542, and a cDNA of a satellite RNA of cucumber mosaic virus are introduced.

4. The method of claim 3, wherein said DNA originating from the virulence region of said pTiBo542 contains virB, virG and virC genes.

5. The method of claim 3, wherein said plasmid containing the DNA originating from the virulence region of said Ti plasmid pTiBo542 is plasmid pTOK162.

F i g .  1

ABBREVIATION : NPT : neomycin phosphotransferase, Ori : replication origin of ColE1

Cos : Cos site of λ phage, BR : border of right, BL : border of left,

TC : tetracycline-resistant gene, SPEC : spetinomycin-resistant gene,

Nos : promoter of nopaline synthase, 73 : cDNA of 73-satRNA,

AP : ampicillin-resistant gene, B : Bam HI, K : Kpn I, S : Sal I, Sa : Sac II,

E : Eco RI, P : Pst I

pYS143
49.5Kb

pYS134
7.6kb

pTOK162
41.9kb

5.2Kb

15.2kb KpnI Fragment

pBR322
Homology

```
           10                  20                  30
   GUUUUGUUUG   AUGGAGAAUU   GCGUAGAGGG

           40                  50                  60
   GUUAUAUCUA   CGUGAGGAUC   CAUCACUCGG

           70                  80                  90
   CGGUGUGGGU   UACCUCCCUG   CUACGGCGGG

          100                 110                 120
   UUGAGUCGAC   GCACCUCGGA   CUGGGGACCG

          130                 140                 150
   CUGGCUUGCG   AGCUAUGUCC   GCUACCCUCA

          160                 170                 180
   GCACUACGCG   CUCAUUUGAG   CCCCCGCUCA

          190                 200                 210
   GUUUGCUAGC   AGAACCCGGC   ACAUGGUUCG

          220                 230                 240
   CCGUUACCAU   GGACUUCGAA   AGAAAAACUC

          250                 260                 270
   UGUUAGGUGG   UAUCGUGGAU   GACGCACGCA

          280                 290                 300
   GGGAGAGGCU   AAGACUUAUG   UUAUGCUGAU

          310                 320                 330
   CUCCGUGAAU   GUCUAUACAU   UCCUCUACAG

   GACCC
```

Fig. 2

Fig. 3

1. Non-transfomed JTM-7

2. Transformant Sat-71

3. Transformant Sat-72

Fig. 4

1. Non-transfomed JTM-7

2. Transformant Sat-71

3. Transformant Sat-72

Fig. 5

Lane 1 : Non-inoculated Transformant Sat-71

Lane 2 : Sat-71 inculated with CMV O strain

Lane 3 : Non-transformed JTM-7 inculated with a mixture of CMV O strain
and T73-sat RNA

ds RNA : Band of double-stranded T73-satRNA

ss RNA : Band of single-stranded T73-satRNA